# EUROPÄISCHE PATENTSCHRIFT

(11) **EP 2 992 906 B1**
(45) Veröffentlichungstag und Bekanntmachung des Hinweises auf die Patenterteilung: **21.02.2024**
(21) Anmeldenummer: 15184159.0
(22) Anmeldetag: 08.09.2015
(51) Int. Cl.: A61L 2/20, B08B 9/20, B67C 3/22

(54) **VERFAHREN ZUR ZWISCHENSTERILISATION MINDESTENS EINER OBERFLÄCHE IN EINEM ISOLATOR EINER ANLAGE ZUR BEHANDLUNG VON BEHÄLTERN**
METHOD FOR INTERIM STERILIZATION OF AT LEAST ONE SURFACE IN AN INSULATOR OF A SYSTEM FOR TREATING CONTAINERS
PROCEDE DE STERILISATION PARTIELLE D'AU MOINS D'UNE SURFACE DANS UN ISOLATEUR D'UNE INSTALLATION DE TRAITEMENT DE RECIPIENTS

(30) Priorität: 08.09.2014 DE 102014112895
(43) Veröffentlichungstag der Anmeldung: 09.03.2016
(73) Patentinhaber: Krones AG, 93073 Neutraubling (DE)
(72) Erfinder: MUELLER, Holger, 93073 Neutraubling (DE); SOELLNER, Juergen, 93073 Neutraubling (DE); DOEBLER, Martin, 93073 Neutraubling (DE)
(74) Vertreter: Nordmeyer, Philipp Werner

(56) Entgegenhaltungen:
- EP-A1- 2 609 938
- EP-A1- 2 711 158
- WO-A1-00/27440
- WO-A1-2008/014991
- JP-A- 2010 189 034
- US-A1- 2010 196 216

## Beschreibung

### Technisches Gebiet

Die vorliegende Erfindung betrifft ein Verfahren zur Zwischensterilisation mindestens einer Oberfläche in einem Isolator einer Anlage zur Behandlung von Behältern bei einer Betriebsunterbrechung, bevorzugt in einer Getränkeabfüllanlage, einer Anlage zum Verschließen von Behältern und/oder einer Anlage zum Umformen von Behältervorformlingen in zu befüllende Behälter.

### Stand der Technik

Die Verfahren zur Sterilisation von Reinräumen bei Lebensmittelverpackungsanlagen lassen sich grob in zwei Technologiegruppen einteilen: Nassaseptikverfahren und Trockenaseptikverfahren. Ein Nassaseptikverfahren geht beispielsweise aus der DE 10 2006 036 475 A1 hervor, in der die Sterilisation dadurch erreicht wird, dass dampf- oder aerosolförmiges Wasserstoffperoxid in den zu sterilisierenden Isolator eingebracht wird, woraufhin sich innerhalb des Isolators ein Kondensationsfilm mit dem Sterilisationsmittel bildet. Das an den jeweiligen Oberflächen kondensierte Wasserstoffperoxid führt die Desinfektion der jeweiligen Oberfläche aus. Das kondensierte Wasserstoffperoxid muss vor Aufnahme der Produktion vollständig aus dem Isolator entfernt werden, um einen Eintrag in das Füllprodukt zu verhindern.

Bei einem Trockenaseptikverfahren wird der Isolator während der Sterilisierung trocken gehalten. Entsprechend wird die Kondensation des verdampften Sterilisationsmittels auf den Oberflächen verhindert, um das Risiko eines Eintragens von Sterilisationsmittel in das Füllprodukt von Vornherein zu minimieren. Ein Verfahren zur Trockenaseptik geht beispielsweise aus der DE 10 2012 108 978 A1 hervor.

Gemäß dem Stand der Technik der Trockenaseptik wird der Isolator folgendermaßen in einen sterilen Zustand gebracht: zunächst findet eine Reinigung der Oberflächen des Isolators über ein Düsensystem statt, welches ein Reinigungsmittel auf die Isolatoroberflächen bringt. Anschließend werden die Oberflächen mit Heißwasser gespült. Nach diesem Reinigungsschritt erfolgt ein Trocknungsschritt, in welchem erhitzte Luft über das Düsensystem und die Lüftungstechnik den Isolator trocknet. Nach erfolgter Trocknung wird in den erhitzten Isolator verdampftes Wasserstoffperoxid eingeblasen, um alle frei zugänglichen Oberflächen zu sterilisieren. Danach befindet sich das System in einem sterilen Zustand und steht für die Produktion bereit.

Das oben genannte Trockenaseptikverfahren kann nur nach einem vorherigen Beenden der Produktion durchgeführt werden, so dass es nicht möglich ist, eine Sterilisierung der Oberflächen des Isolators bei einer Öffnung des Isolators zur Durchführung eines notwendigen Eingriffs, etwa zur Behebung kleinerer Störungen, wieder zu sterilisieren, ohne die Produktion zu unterbrechen. Bei einer solchen störungsbedingten Betriebsunterbrechung muss entsprechend die Produktion beendet werden und dann der oben beschriebene Reinigungszyklus durchlaufen werden.

Die US 2010/0196216 A1 beschreibt eine Vorrichtung zum Zuführen eines Sterilisationsmittels in einen Isolator. Die EP 2 711 158 A1 beschreibt ein Verfahren zum Herstellen von Getränkebehältnissen und zum Auswechseln von Blasformteilen. Die WO 2008/014991 A1 beschreibt ein Verfahren zur

Sterilisation von Reinräumen für die Behandlung und/oder das Füllen und Verschließen von Behältern. Die EP 2 609 938 A1 beschreibt einen Isolator mit einer Sterilisationsmittel-Reduktionseinheit. Die WO 00/27440 A1 beschreibt eine Vorrichtung und ein Verfahren zum Sterilisieren von Schraubkappen.

Die JP 2010-189034 A beschreibt ein Verfahren zur Sterilisation einer Oberfläche in einem Isolator einer Getränkeabfüllanlage.

### Darstellung der Erfindung

Eine Aufgabe der vorliegenden Erfindung besteht daher darin, eine Zwischensterilisation bei einer Trockenaseptikanlage zu ermöglichen, ohne den Aufbau der Anlage wesentlich zu verkomplizieren und bei geringen Investitions- und Wartungskosten.

Gelöst wird die Aufgabe mit einem Verfahren mit den Merkmalen des Anspruchs 1. Vorteilhafte Weiterbildungen folgen aus den Unteransprüchen, der folgenden Darstellung der Erfindung sowie der Beschreibung bevorzugter Ausführungsbeispiele.

Entsprechend wird ein Verfahren zur Zwischensterilisierung mindestens einer Oberfläche in einem Isolator einer Anlage zur Behandlung von Behältern bei einer Betriebsunterbrechung, bevorzugt in einer Getränkeabfüllanlage, vorgeschlagen, wobei das Verfahren die Schritte des Aufbringens eines Temperierfluids auf mindestens eine Oberfläche des Isolators, um diese zu temperieren, des wenigstens teilweisen Entfernens der Temperierfluids aus dem Isolator, des Beaufschlagens der Oberfläche mit einem Sterilisationsmittel, und des Entfernens des Sterilisationsmittels nach einer vorgegebenen Einwirkzeit vorschlägt.

Das Verfahren betrifft entsprechend eine Zwischensterilisation einer Oberfläche eines Isolators einer Anlage zur Behandlung von Behältern bei einer Betriebsunterbrechung. Mit *"Zwischensterilisation"* ist gemeint, dass diese als zusätzliches Programm zur eigentlichen, initialen Reinigung/Sterilisation der Anlage vorgesehen ist. Die Zwischensterilisation kann nach einer Öffnung des Isolators zur Durchführung eines Eingriffs in die Anlage angewählt werden, um das System wieder in einen sterilen Zustand zu bringen. Eine vorangestellte Reinigung des Systems ist in diesem Zwischensterilisations-Programm nicht notwendig und ist vorzugsweise auch nicht vorgesehen. Die Zwischensterilisation kann somit durchgeführt werden, ohne dass ein vollständiger Stopp der Produktion, der einen Neustart der Anlage und somit eine Reduktion der Ausbringung mit sich bringen würde, erforderlich ist.

Gemäß dem Verfahren zur Zwischensterilisation wird ein Temperierfluid, vorzugsweise erhitztes Wasser, auf die frei zugänglichen Oberflächen im Isolator eingebracht, wodurch der Isolator, insbesondere die zu sterilisierenden Oberflächen des Isolators, temperiert wird. Das Temperierfluid weist dazu bevorzugt eine Temperatur auf, die größer als die Temperatur des Isolators vor dem Einbringen des Temperierfluids ist. Das Temperierfluid wird anschließend teilweise oder vollständig aus dem Isolator entfernt. Dies geschieht beispielweise durch eine geeignete Konstruktion des Isolators, die ein rasches Ablaufen der Flüssigkeit ermöglicht. Ferner erfolgt die Trocknung durch Verdunstung, die dadurch gefördert wird, dass das Temperierfluid temperiert ist und entsprechend die Oberflächen aufgeheizt sind. Es ist darüber hinaus möglich, weitere Maßnahmen zur Trocknung vorzunehmen, beispielsweise ein aktives oder passives Durchlüften des Isolators. Anschließend an die Aufheizung der Oberflächen des Isolators wird ein trockenes Sterilisationsmittel, vorzugsweise verdampftes Wasserstoffperoxid, in den Isolator eingebracht und nach einer vorgegebenen Einwirkzeit wieder aus dem Isolator entfernt. Danach kann die unterbrochene Produktion sofort wieder aufgenommen werden.

Bevorzugt ist das Sterilisationsmittel ein trockenes Sterilisationsmittel, bevorzugt verdampftes und/oder vernebeltes Wasserstoffperoxid. Auf diese Weise kann auf das bereits in der Anlage in der Trockenaseptik vorliegende Sterilisationsmittel, welches auch zur Initialisierung der Anlage verwendet wird, zurückgegriffen werden. Durch den Eintrag des trockenen Sterilisationsmittels wird so sichergestellt, dass bei der Zwischensterilisation kein Sterilisationsmittel im Isolator kondensiert und damit auch nicht in das Füllprodukt gelangen kann, so dass bei einer nachfolgenden Wiederaufnahme der Produktion kein Risiko des Eintrags von Sterilisationsmittel in das Füllprodukt besteht.

Das oben dargelegte Verfahren lässt sich ohne erheblichen Nachrüstungsaufwand mit bereits bestehenden Trockenaseptikanlagen durchführen. Dies geschieht, indem die bereits vorhandenen Reinigungsdüsen, die zur initialen Reinigung, Durchlüftung und/oder Sterilisation der Anlage vorgesehen sind, auch zum Einbringen des Temperierfluids und des trockenen Sterilisationsmittels verwendet werden. Somit sind keine oder nur wenige zusätzliche konstruktive Maßnahmen erforderlich, um das Verfahren zur Zwischendesinfektion durchführen zu können. Eine Nachrüstung ist im Wesentlichen durch eine Erweiterung der Steuerungsvorrichtung der Anlage möglich.

Um eine Kondensation des Sterilisationsmittels im Isolator zu vermeiden, wird die mindestens eine Oberfläche des Isolators mittels des Temperierfluids auf eine Temperatur gebracht, die oberhalb des Kondensationspunkts des Sterilisationsmittels liegt. Das Temperierfluid kann bevorzugt eine Temperatur im Bereich von 70°C bis 95°C, vorzugsweise etwa 80°C, aufweisen.

Um während des Temperieren der Oberflächen auch einen mechanischen Reinigungseffekt bereitstellen zu können, wird das Temperierfluid bevorzugt mit einem Druck im Bereich von 2 bar und 4 bar, vorzugsweise etwa 3 bar, auf die Oberflächen aufgebracht.

Eine Ausbildung des Verfahrens ohne eine Änderung des Aufbaus des Isolators lässt sich erreichen, indem der Isolator Außenreinigungsdüsen zur Beaufschlagung der mindestens Oberfläche mit einem Reinigungsfluid aufweist und das Temperierfluid und/oder das Sterilisationsmittel durch eine oder mehrere der Außenreinigungsdüsen auf die Oberfläche aufgebracht wird.

Bevorzugt weist der Isolator Komponenten zur Befüllung von Behältern mit flüssigen Lebensmitteln auf. Mit anderen Worten ist der Isolator zur Aufnahme einer Füllvorrichtung für die Behälter versehen.

Eine Zwischenreinigung ist bevorzugt dann vorgesehen, wenn vor dem Aufbringen des Temperierfluids eine Unterbrechung des Normalbetriebs der Anlage sowie eine Öffnung und ein nachfolgendes Verschließen des Isolators stattfindet, und nach Entfernen des Sterilisationsmittels der Normalbetrieb der Anlage wieder aufgenommen wird.

Eine besonders einfache Ausbildung des Verfahrens lässt sich bevorzugt erreichen, indem das Temperierfluid Wasser, bevorzugt steriles Wasser, ist.

Eine vollständige Sterilisierung der Anlage zur Behandlung von Behältern lässt sich bevorzugt erreichen, wenn alle sich im Isolator befindlichen, frei zugänglichen Oberflächen der Zwischensterilisierung unterzogen werden. Damit ist es möglich, den durch eine etwaige Öffnung des Isolators während einer Betriebsunterbrechung erreichten nicht sterilen Zustand des Isolators durch die Zwischensterilisierung wieder herzustellen und entsprechend sicher zu stellen, dass nach der Zwischensterilisierung der Normalbetrieb wieder in einer vorschriftsmäßig sterilen Anlage aufgenommen werden kann.

Unter einer frei zugänglichen Oberfläche wird eine solche verstanden, die innerhalb des Isolators angeordnet ist und ohne den Abbau von Komponenten oder die Öffnung von Ventilen zugänglich ist. Genau diese Oberflächen sind bei einer kurzzeitigen Öffnung des Isolators bei einer Betriebsunterbrechung auch gefährdet, in einen nicht-sterilen Zustand zu gelangen. Etwaige nicht frei zugängliche Oberflächen wie beispielsweise die in den einzelnen Komponenten der Anlage zur Behälterbehandlung vorgesehenen Kanäle zum Führen des Füllprodukts oder anderer Medien sind hingegen bei einer Betriebsunterbrechung nicht gefährdet, in einen nicht-sterilen Zustand zu gelangen, da sie mit den jeweiligen Medien oder Füllprodukt gefüllt sind und entsprechend mit der nicht-sterilen Luft, die bei der Öffnung in den Isolator strömen kann, nicht in Kontakt kommen.

Durch die Mischung aus mechanischem, thermischem und chemischem Eintrag in den Isolator wird eine große Bandbreite an Keimen abgetötet. Das System muss daher nicht neu gereinigt und vollständig sterilisiert werden, sollte bei einer Betriebsunterbrechung ein Eingriff in den Isolator erforderlich gewesen sein. Damit kann auf einen vollständigen Stopp der Anlage verzichtet werden. Die Produktion wird lediglich für den Zeitraum des Eingriffs in den Isolator und der Zwischensterilisation unterbrochen, wobei in bestimmten Fällen sogar auf eine solche zwischenzeitliche Unterbrechung verzichtet werden kann. Das Temperierfluid dient bei der Zwischensterilisation primär zur Erhitzung der zu desinfizierenden Oberflächen. Aufgrund der so erzielten Temperatur der Oberfläche wird eine Kondensation des Sterilisationsmittels im Isolator vermieden, wodurch das Zwischensterilisationsverfahren der Trockenaseptik zugeordnet wird. Durch Einbringen der temperierten Flüssigkeit werden die zu sterilisierenden Oberflächen des Isolators rasch erhitzt, schneller als beispielsweise mit Heißluft. Ferner kann auf zusätzliche konstruktive Maßnahmen, wie beispielsweise Elektroheizer oder dergleichen, verzichtet werden. Vorzugsweise ist das trockene Sterilisationsmittel verdampftes Wasserstoffperoxid, da dieses für die Trockenaseptik und insbesondere zur Anwendung im Lebensmittelbereich geeignet ist.

Vorzugsweise werden das Temperierfluid und/oder das Sterilisationsmittel durch eine oder mehrere Außenreinigungsdüsen in den Isolator eingebracht. Die Außenreinigungsdüsen sind vorgesehen, um bei einer Initialisierung der Anlage, also zur Grundreinigung, -trocknung und -sterilisation des Systems, Reinigungsmittel, Trockenluft und Desinfektionsmittel einzubringen. Diese Systemkomponenten werden nun auf synergetische Weise zur Zwischensterilisation genutzt.

Vorzugsweise findet die Zwischensterilisation während des Normalbetriebs statt. Das heißt, die Zwischensterilisation erfolgt, ohne dass ein vollständiger Stopp der Anlage durchgeführt wird. Die Produktion wird allenfalls nur kurz, nämlich für den Zeitraum der Zwischensterilisation und des Ereignisses, das die Zwischensterilisation erforderlich macht, angehalten. Ein solches Anhalten der Produktion unterscheidet sich von einem vollständigen Stopp dadurch, dass die Anlage nicht neu initialisiert werden muss, dass auf eine Komplettreinigung der Anlage verzichtet werden kann, Teile der Anlage in Betrieb bleiben können und die Medien und das Füllprodukt in den jeweiligen Kanälen verbleiben können. Vorzugsweise ist der Zwischensterilisation somit keine Reinigung des Isolators vorangestellt, die über die vernachlässigbare Reinigungswirkung durch die Temperierfluid hinausgeht.

Die oben dargelegten Verfahren zur Zwischensterilisation finden im Rahmen des Betriebs einer Anlage zur Behandlung von Behältern statt. Dieser Betrieb umfasst als weitere Schritte beispielsweise eine initiale Reinigung, Trocknung und Sterilisation der Anlage und allgemein eine Behandlung von Behältern, die etwa eine Etikettierung, ein Verschließen, ein Abfüllen und/oder ein Blasformen von Behältervorformlingen in Behälter umfassen kann.

Vorzugsweise ist der Isolator Bestandteil einer Anlage zum Befüllen von Behältern mit flüssigen Lebensmitteln, zur Etikettierung von Behältern, zum Verschließen von Behältern und/oder zum Blasumformen von Behältervorformlingen in Behälter. Besonders bevorzugt ist der Isolator Bestandteil einer Lebensmittelabfüllanlage. Hierbei umfassen die abzufüllenden Lebensmittel sowohl niedrig- als auch hochviskose und pastöse Flüssigkeiten, wie auch mehrphasige Flüssigkeiten und solche mit Partikeln. Getränke aller Art sind ebenso einbezogen wie Joghurt, Konfitüre, Gelee und andere Lebensmittel. Die zu befüllenden Behälter sind vorzugsweise Kunststoffbehälter, etwa PET-Flaschen.

Insbesondere wird die oben geschilderte Aufgabe auch durch ein Verfahren zum Betrieb einer Anlage zur Behandlung von Behältern, die einen Isolator aufweist, gelöst wobei das Verfahren die Schritte der Reinigung und Sterilisation der Anlage, der Aufnahme des Betriebs der Anlage im Normalbetrieb zur Behandlung von Behältern, bevorzugt zur Befüllung der Behälter mit einem Füllprodukt, des Unterbrechens des Normalbetriebs sowie des Öffnens und nachfolgenden Verschließens des Isolators, der Zwischensterilisation des Isolators mit einem der oben beschriebenen Verfahren, und die Wiederaufnahme des Normalbetriebs der Anlage umfasst. Dabei weist die Reinigung und Sterilisation der Anlage vor der Aufnahme des Normalbetriebs bevorzugt die Schritte der Reinigung der im Isolator aufgenommenen, frei zugänglichen Oberflächen durch das Einbringen eines Reinigungsmittels, des Spülens der Oberflächen mit Heißwasser, des Trocknens der Oberflächen durch Einbringen erhitzter, trockener Luft, und des Beaufschlagens der gereinigten und getrockneten Oberflächen mit einem Sterilisationsmittel auf. Wenngleich die vorliegende Erfindung besonders bevorzugt bei der Abfüllung flüssiger Lebensmitteln zur Anwendung kommt, versteht es sich, dass die Erfindung auch in anderen Bereichen umgesetzt werden kann. Darüber hinaus sind weitere Vorteile und Merkmale der vorliegenden Erfindung aus der folgenden Beschreibung bevorzugter Ausführungsbeispiele ersichtlich.

### Kurze Beschreibung der Figur

Die folgende Beschreibung der bevorzugten Ausführungsbeispiele erfolgt dabei unter Bezugnahme auf die begleitende Zeichnung.

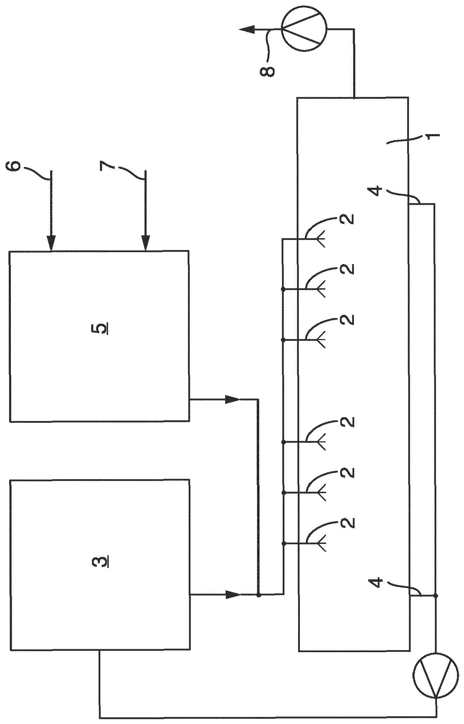

Die Figur 1 zeigt schematisch eine Anlage, die zur Zwischensterilisation geeignet ist und anhand welcher ein mögliches Zwischensterilisationsverfahren erläutert wird.

### Detaillierte Beschreibung bevorzugter Ausführungsbeispiele

Die Figur zeigt schematisch eine Anlage zur Behandlung von Behältern im Lebensmittelbereich, wobei die Anlage als aseptische Anlage, insbesondere trockenaseptische Anlage ausgebildet ist. Die Behandlung umfasst insbesondere die Abfüllung flüssiger oder viskoser Lebensmittel wie beispielsweise Getränke in dafür geeignete Behälter.

Die Anlage weist einen sehr schematisch angedeuteten Isolator 1 auf, der auch als Reinraum bezeichnet wird, und der einen gegenüber der Umgebung im Wesentlichen abgeschlossenen Raum bereitstellt. Hierzu wird der Isolator 1 üblicherweise durch Isolatorwände aus einem Edelstahl bereitgestellt, die den abgeschlossenen Raum umgeben und damit ausbilden.

Im Isolator 1 wird eine definierte und keimarme Atmosphäre bereitgestellt, um eine aseptische Behandlung der Behälter zu ermöglichen. Dies ist beispielsweise bei der Abfüllung leicht verderblicher Lebensmittel oder von Lebensmitteln, welche eine lange Lagerzeit (shelf life) erreichen sollen, von Bedeutung. In einer solchen aseptischen Behälterbehandlungsanlage werden beispielsweise Milchprodukte abgefüllt.

In dem Isolator 1 sind die einzelnen Komponenten zur Behandlung der Behälter aufgenommen. Im Isolator 1 sind beispielsweise ein Füllerkarussell zum kontinuierlichen Befüllen von Behältern, Transportsterne zum Transportieren der zu befüllenden und der befüllten Behälter und ein Verschließer zum Verschließen der befüllten Behälter mit einem Behälterverschluss vorgesehen. In dem Isolator 1 kann auch eine Behälterblasmaschine vorgesehen sein, mittels welcher zu befüllende Behälter aus Behälterrohlingen mittels Blasen oder Streckblasen hergestellt werden. Die einzelnen Komponenten können in einem durch ein einziges Isolatorgehäuse ausgebildeten Isolator 1 oder in einem durch mehrere aneinandergereihte Isolatorgehäuse ausgebildeten Isolator 1 aufgenommen sein. Über entsprechend ausgebildete Schleusen können im Normalbetrieb der Anlage zu befüllende Behälter oder Behälterrohlinge in den Isolator 1 eingebracht werden und befüllte und verschlossene Behälter den Isolator 1 verlassen. Bei den Schleusen handelt es sich häufig um Überdruckschleusen, bei welchen ein konstanter Strom von in den Isolator 1 eingeführter Sterilluft durch die Schleusen in die Umgebung austritt, so dass Keime nicht entgegen dem Sterilluftstrom in den Isolator 1 eindringen können.

Im Isolator 1, beispielsweise in einer Isolatorwand, sind weiterhin Serviceöffnungen vorgesehen, über welche ein Bediener bei etwaigen Betriebsstörungen oder zur Wartung von Komponenten in den Isolator 1 eingreifen kann. Bei einer Öffnung der Serviceöffnungen geht die Sterilität der Anlage verloren und muss nach einer Betriebsstörung wieder erneut hergestellt werden.

Um vor der Aufnahme des Normalbetriebs der Behälterbehandlung den Innenraum des Isolators 1 zu reinigen und zu sterilisieren sind im Isolator 1 schematisch angedeutete Reinigungsdüsen 2 vorgesehen, die zum Einbringen eines Reinigungsmittels, von Sterilluft und/oder eines Sterilisationsmittels während der initialen Reinigung und Sterilisation vorgesehen sind. Mittels der Reinigungsdüsen 2 können die im Isolator 1 aufgenommenen, frei zugänglichen Oberflächen entsprechend mit den Reinigungs- und Sterilisierungsmedien beaufschlagt werden, um eine Reinigung und Sterilisierung zu ermöglichen. Dabei sind die Reinigungsdüsen 2 als sogenannte Außenreinigungsdüsen vorgesehen, welche die im Isolator 1 frei zugänglichen Oberflächen mit den Reinigungs- und Sterilisationsmedien beaufschlagen. Damit werden nicht nur die frei zugänglichen Oberflächen der in dem Isolator 1 aufgenommenen Komponenten zur Behälterbehandlung, sondern auch die Innenoberflächen der Isolatorwände mit den Reinigungs- und Sterilisationsmedien beaufschlagt.

Eine Reinigung und Sterilisierung der inneren Oberflächen, beispielsweise der Oberflächen der produktführenden Kanäle oder von medienführenden Kanälen, wird durch ein separates Reinigungs- und Sterilisationsverfahren erreicht, welches gleichzeitig mit der Reinigung und Sterilisierung der frei zugänglichen Oberflächen, oder aber zu einem anderen Zeitpunkt durchgeführt werden kann. Die initiale Reinigung und Sterilisierung der inneren Oberflächen ist prinzipiell bekannt.

Die Reinigungsdüsen 2 stehen mit einem CIP-Modul 3 in Verbindung, welches die entsprechenden Reinigungsmedien und Sterilisationsmedien in einer vorgegebenen Temperatur und Konzentration sowie einem vorgegebenen Druck bereitstellt. In dem CIP-Modul 3 können die jeweiligen Medien auch wieder aufgefrischt oder erneuert werden, wenn die Reinigung und Sterilisierung in einem Kreislaufverfahren stattfindet. "CIP" steht für "Cleaning In Place" und bedeutet, dass die Anlage so ausgelegt ist, dass eine Reinigung derselben möglich ist, ohne diese teilweise oder ganz auseinanderzunehmen.

Um eine Reinigung und Sterilisierung bei geschlossenem Isolator 1 durchführen zu können und die jeweiligen Medien wieder aus dem Isolator 1 entfernen zu können, weist die Vorrichtung ein Ablaufsystem 4 der in den Isolator 1 eingebrachten Medien auf. Die Wege zur Abführung der Medien sind in der Figur nur schematisch als Ablaufsystem 4 dargestellt.

Zusätzlich zum CIP-Modul 3 sind die Reinigungsdüsen 2 außerdem mit einem Wasserstoffperoxid-Verdampfer 5 verbunden, der verdampftes Wasserstoffperoxid herstellt und den Reinigungsdüsen 2 zuführt. Dazu wird dem Wasserstoffperoxid-Verdampfer 5 über eine Leitung 6 Wasserstoffperoxid-Konzentrat und über eine Leitung 7 Prozessluft zugeführt. Der Wasserstoffperoxid-Verdampfer 5 ist somit zur Herstellung und Zuführung des Sterilisationsmittels in den Isolator 1 verantwortlich. Das Absaugen gasförmiger Komponenten aus dem Isolator 1 erfolgt über die Abluftleitung 8.

Bei der Initialisierung der Anlage wird der Isolator 1 wie folgt in den gereinigten und sterilen Zustand gebracht: zunächst findet eine Reinigung der Oberflächen des Isolators 1 über die Reinigungsdüsen 2 statt, indem ein Reinigungsmittel von dem CIP-Modul 3 in der vorgesehenen Temperatur, Konzentration und Menge sowie unter dem vorgesehenen Druck bereitgestellt und auf die frei zugänglichen Oberflächen im Isolator 1 eingebracht wird. Danach werden die Oberflächen des Isolators 1 mit Heißwasser gespült, welches ebenfalls durch das CIP-Modul 3 bereitgestellt wird und über die Reinigungsdüsen 2 auf die Oberflächen gebracht wird. Das Reinigungsmittel und das Heißwasser werden nacheinander über das Ablaufsystem 4 aus dem Isolator 1 entfernt.

Danach erfolgt ein Trocknungsschritt, in welchem erhitzte Luft, bevorzugt Sterilluft, mittels des CIP-Moduls 3 bereitgestellt wird und über die Reinigungsdüsen 2 in den Isolator 1 eingebracht wird. Durch die heiße Luft und gegebenenfalls weitere Maßnahmen der Lüftungstechnik, die in der Figur nicht dargestellt sind, werden die Oberflächen des Isolators 1 getrocknet.

Nach der Trocknung wird über den Wasserstoffperoxid-Verdampfer 5 und die Reinigungsdüsen 2 verdampftes Wasserstoffperoxid in den Isolator 1 eingebracht, wodurch die Oberflächen darin sterilisiert werden. Nach erfolgter Sterilisation und Entfernung des Wasserstoffperoxids aus dem Isolator befindet sich das System in einem sterilen Zustand und steht für die Produktion im Normalbetrieb bereit.

Ist während der Produktion eine Unterbrechung des Normalbetriebs mit einem Eingriff in den Isolator 1 erforderlich, etwa um eine Störung zu beheben, dann ist nicht gewährleistet, dass sich der Isolator 1 danach noch in einem sterilen beziehungsweise aseptischen Zustand befindet. Ein Eingriff kann beispielsweise durch die Öffnung und das Schließen des Isolators 1, beispielsweise durch Öffnen und Schließen einer Serviceöffnung, durchgeführt werden.

Nach dem erneuten Schließen des Isolators muss entsprechend eine Zwischensterilisation durchgeführt werden, die wie folgt vonstattengeht: zunächst wird ein Temperierfluid in Form von sterilem temperiertem Wasser, welches durch das CIP-Modul 3 bereitgestellt wird und welches eine Temperatur von etwa 80°C aufweist, über die Reinigungsdüsen 2 auf die frei zugänglichen Oberflächen in dem Isolator 1 aufgebracht. Durch diesen Schritt werden die Oberflächen aufgeheizt.

Unter einem Aufheizen der Oberflächen wird verstanden, dass das die Oberflächen ausbildende Material, beispielsweise Edelstahl, aufgeheizt wird und damit entsprechend auch die Oberflächen selbst eine erhöhte Temperatur annehmen.

Vorzugsweise wird das temperierte Wasser mit einem Druck von etwa 3 bar auf die Oberflächen aufgebracht, um auch eine gewisse mechanische Reinigungswirkung bereit zu stellen. Das Temperierfluid wird über das Ablaufsystem 4 abgezogen. Ein schnelles Abtrocknen der Oberflächen ist durch das Ablaufsystem 4 und die hohe Temperatur des Wassers sowie der entsprechend aufgeheizten Oberflächen gegeben. Nachdem die Oberflächen abgetrocknet sind, wird über die Reinigungsdüsen 2 verdampftes Wasserstoffperoxid in den Isolator 1 eingebracht, wodurch die zu desinfizierenden Oberflächen sterilisiert werden. Dadurch, dass die Oberflächen erhitzt sind, kann das Wasserstoffperoxid nicht an den Oberflächen kondensieren, so dass die Oberflächen trocken bleiben. Nach dem Abziehen des Sterilisationsmittels aus dem Isolator, beispielsweise durch eine Spülung mit Sterilluft, kann der Normalbetrieb daher ohne neuerliche vollständige Reinigung und Sterilisierung beziehungsweise Initialisierung fortgesetzt werden.

Die Zwischensterilisation erfolgt bevorzugt unter Zuhilfenahme der bereits vorhandenen Komponenten, insbesondere des CIP-Moduls 3, des Wasserstoffperoxid-Verdampfers 5 und den damit verbundenen Reinigungsdüsen 2. Eine Nachrüstung des Systems ist somit leicht möglich, ohne dass sich die Anlage erheblich verkompliziert. Durch die Mischung aus thermischer, mechanischer und chemischer Behandlung der frei zugänglichen Oberflächen im Isolator 1 wird eine große Bandbreite von Keimen abgetötet und der Normalbetrieb kann auch nach einer Betriebsunterbrechung wieder aufgenommen werden.

Bei einer Unterbrechung des Normalbetriebs sind die produktführenden Kanäle sowie die medienführenden Kanäle noch mit den jeweiligen Medien gefüllt, so dass eine Reinigung und Desinfizierung nicht notwendig ist. In diesem Zusammenhang kann es aber sinnvoll sein, die ersten nach der Unterbrechung des Normalbetriebs befüllten Behälter zu verwerfen.

### Bezugszeichenliste

- 1: Isolator
- 2: Reinigungsdüsen
- 3: CIP-Modul
- 4: Ablaufsystem
- 5: Wasserstoffperoxid-Verdampfer
- 6: Zufuhr von Wasserstoffperoxid-Konzentrat
- 7: Zufuhr von Prozessluft
- 8: Abluft

## Patentansprüche

1. Verfahren zur Zwischensterilisierung mindestens einer Oberfläche in einem Isolator (1) einer Anlage zur Behandlung von Behältern bei einer Betriebsunterbrechung, bevorzugt in einer Getränkeabfüllanlage, wobei das Verfahren die Schritte aufweist:
Aufbringen eines Temperierfluids auf mindestens eine Oberfläche des Isolators (1), um diese zu temperieren,
wenigstens teilweises Entfernen des Temperierfluids aus dem Isolator (1),
Beaufschlagen der Oberfläche mit einem Sterilisationsmittel, und
Entfernen des Sterilisationsmittels nach einer vorgegebenen Einwirkzeit, **dadurch gekennzeichnet, dass**
die mindestens eine Oberfläche des Isolators (1) mittels des Temperierfluids auf eine Temperatur gebracht wird, die oberhalb des Kondensationspunkts des Sterilisationsmittels liegt.

2. Verfahren nach Anspruch 1, **dadurch gekennzeichnet, dass** das Sterilisationsmittel ein trockenes Sterilisationsmittel ist, bevorzugt verdampftes und/oder vernebeltes Wasserstoffperoxid.

3. Verfahren nach einem der vorhergehenden Ansprüche, **dadurch gekennzeichnet, dass** das Temperierfluid eine Temperatur im Bereich von 70°C bis 95°C, vorzugsweise etwa 80°C, aufweist.

4. Verfahren nach einem der vorhergehenden Ansprüche, **dadurch gekennzeichnet, dass** das Temperierfluid mit einem Druck im Bereich von 2 bar und 4 bar, vorzugsweise etwa 3 bar, auf die Oberfläche aufgebracht wird.

5. Verfahren nach einem der vorhergehenden Ansprüche, **dadurch gekennzeichnet, dass** der Isolator (1) Außenreinigungsdüsen (2) zur Beaufschlagung der Oberfläche mit einem Reinigungsfluid aufweist und das Temperierfluid und/oder das Sterilisationsmittel durch eine oder mehrere der Außenreinigungsdüsen (2) auf die Oberfläche aufgebracht wird.

6. Verfahren nach einem der vorhergehenden Ansprüche, **dadurch gekennzeichnet, dass** der Isolator (1) Komponenten zur Befüllung von Behältern mit flüssigen Lebensmitteln aufweist.

7. Verfahren nach einem der vorhergehenden Ansprüche, **dadurch gekennzeichnet, dass** vor dem Aufbringen des Temperierfluids eine Unterbrechung des Normalbetriebs der Anlage sowie eine Öffnung und ein nachfolgendes Verschließen des Isolators (1) stattfindet, und nach Entfernen des Sterilisationsmittels der Normalbetrieb der Anlage wieder aufgenommen wird.

8. Verfahren nach einem der vorhergehenden Ansprüche, **dadurch gekennzeichnet, dass** das Temperierfluid Wasser, bevorzugt steriles Wasser, ist.

9. Verfahren nach einem der vorhergehenden Ansprüche, **dadurch gekennzeichnet, dass** alle sich im Isolator (1) befindlichen, frei zugänglichen Oberflächen der Zwischensterilisierung unterzogen werden.

10. Verfahren zum Betrieb einer Anlage zur Behandlung von Behältern, die einen Isolator (1) aufweist, wobei das Verfahren aufweist:
Reinigung und Sterilisation der Anlage,
Aufnahme des Betriebs der Anlage im Normalbetrieb zur Behandlung von Behältern, bevorzugt zur Befüllung der Behälter mit einem Füllprodukt,
Unterbrechen des Normalbetriebs sowie Öffnen und nachfolgendes Verschließen des Isolators,
Zwischensterilisation des Isolators (1) mit einem Verfahren nach einem der vorhergehenden Ansprüche, und
Wiederaufnahme des Normalbetriebs der Anlage.

11. Verfahren nach Anspruch 10, **dadurch gekennzeichnet, dass** die Reinigung und Sterilisation der Anlage vor der Aufnahme des Normalbetriebs folgende Schritte aufweist:
Reinigung der im Isolator (1) aufgenommenen, frei zugänglichen Oberflächen durch das Einbringen eines Reinigungsmittels,
Spülen der Oberflächen mit Heißwasser,
Trocknen der Oberflächen durch Einbringen erhitzter, trockener Luft, und
Beaufschlagen der gereinigten und getrockneten Oberflächen mit einem Sterilisationsmittel.

## Claims

1. Method for interim sterilisation of at least one surface in an insulator (1) of a plant for treating containers during an interruption in operation, preferably in a beverage bottling plant, wherein the method has the steps of:
applying a tempering fluid to at least one surface of the insulator (1) in order to temper said surface,
at least partially removing the tempering fluid from the insulator (1),
treating the surface with a sterilising agent, and
removing the sterilising agent after a specified exposure time, **characterised in that** the at least one surface of the insulator (1) is brought by means of the tempering fluid to a temperature which is above the condensation point of the sterilising agent.

2. Method according to claim 1, **characterised in that** the sterilising agent is a dry sterilising agent, preferably vaporised and/or nebulised hydrogen peroxide.

3. Method according to any one of the preceding claims, **characterised in that** the tempering fluid has a temperature in the range of 70°C to 95°C, preferably about 80°C.

4. Method according to any one of the preceding claims, **characterised in that** the tempering fluid is applied to the surface at a pressure in the range of 2 bar and 4 bar, preferably about 3 bar.

5. Method according to any one of the preceding claims, **characterised in that** the insulator (1) has external cleaning nozzles (2) for treating the surface with a cleaning fluid and the tempering fluid and/or the sterilising agent is applied to the surface through one or more of the external cleaning nozzles (2).

6. Method according to any one of the preceding claims, **characterised in that** the insulator (1) has components for filling containers with liquid foodstuffs.

7. Method according to any one of the preceding claims, **characterised in that,** before the application of the tempering fluid, the normal operating mode of the plant is interrupted and the insulator (1) is opened and subsequently sealed, and the normal operating mode of the plant is resumed after the sterilising agent has been removed.

8. Method according to any one of the preceding claims, **characterised in that** the tempering fluid is water, preferably sterile water.

9. Method according to any one of the preceding claims, **characterised in that** all freely accessible surfaces located in the insulator (1) are subjected to interim sterilisation.

10. Method for operating a plant for treating containers, which plant has an insulator (1), wherein the method has the steps of:
cleaning and sterilising the plant,
starting operation of the plant in the normal operating mode to treat containers, preferably to fill the containers with a fill product,
interrupting the normal operating mode and opening and subsequently sealing the insulator,
interim sterilisation of the insulator (1) by a method according to any one of the preceding claims, and
resuming the normal operating mode of the plant.

11. Method according to claim 10, **characterised in that** the cleaning and sterilisation of the plant before starting the normal operating mode has the following steps:
cleaning the freely accessible surfaces accommodated in the insulator (1) by introducing a cleaning agent,
rinsing the surfaces with hot water,
drying the surfaces by introducing heated, dry air, and
treating the cleaned and dried surfaces with a sterilising agent.

## Revendications

1. Procédé de stérilisation intermédiaire au moins d'une surface dans un isolateur (1) d'une installation de traitement de récipients lors d'une interruption de fonctionnement, de préférence dans une installation de remplissage de boissons, dans lequel le procédé présente les étapes suivantes :
l'application d'un fluide de thermorégulation sur au moins une surface de l'isolateur (1) afin de la thermoréguler,
le retrait au moins partiel du fluide de thermorégulation de l'isolateur (1),
l'alimentation de la surface en un moyen de stérilisation, et
le retrait du moyen de stérilisation après un temps d'action prédéfini, **caractérisé en ce que** l'au moins une surface de l'isolateur (1) est amenée à une température qui se situe au-dessus du point de condensation du moyen de stérilisation, au moyen du fluide de thermorégulation.

2. Procédé selon la revendication 1, **caractérisé en ce que** le moyen de stérilisation est un moyen de stérilisation sec, de préférence du peroxyde d'hydrogène vaporisé et/ou nébulisé.

3. Procédé selon l'une quelconque des revendications précédentes, **caractérisé en ce que** le fluide de thermorégulation présente une température dans la plage de 70 °C à 95 °C, de préférence environ de 80 °C.

4. Procédé selon l'une quelconque des revendications précédentes, **caractérisé en ce que** le fluide de thermorégulation est appliqué à une pression dans la plage de 2 bars à 4 bars, de préférence environ de 3 bars, sur la surface.

5. Procédé selon l'une quelconque des revendications précédentes, **caractérisé en ce que** l'isolateur (1) présente des buses de nettoyage extérieur (2) pour l'alimentation de la surface en un fluide de nettoyage et le fluide de thermorégulation et/ou le moyen de stérilisation est appliqué par une ou plusieurs des buses de nettoyage extérieur (2) sur la surface.

6. Procédé selon l'une quelconque des revendications précédentes, **caractérisé en ce que**l'isolateur (1) présente des composants pour le remplissage de récipients avec des aliments liquides.

7. Procédé selon l'une quelconque des revendications précédentes, **caractérisé en ce qu'** avant l'application du fluide de thermorégulation, une interruption du fonctionnement normal de l'installation ainsi qu'une ouverture et une fermeture suivante de l'isolateur (1) ont lieu, et après le retrait du moyen de stérilisation, le fonctionnement normal de l'installation est repris.

8. Procédé selon l'une quelconque des revendications précédentes, **caractérisé en ce que** le fluide de thermorégulation est de l'eau, de préférence de l'eau stérile.

9. Procédé selon l'une quelconque des revendications précédentes, **caractérisé en ce que** toutes les surfaces librement accessibles, se trouvant dans l'isolateur (1) sont soumises à la stérilisation intermédiaire.

10. Procédé de fonctionnement d'une installation de traitement de récipients qui présente un isolateur (1), dans lequel le procédé présente :
le nettoyage et la stérilisation de l'installation,
l'entrée en fonctionnement de l'installation en fonctionnement normal pour le traitement de récipients, de préférence pour le remplissage des récipients avec un produit de remplissage,
l'interruption du fonctionnement normal ainsi que l'ouverture et la fermeture suivante de l'isolateur,
la stérilisation intermédiaire de l'isolateur (1) avec un procédé selon l'une quelconque des revendications précédentes, et
la reprise du fonctionnement normal de l'installation.

11. Procédé selon la revendication 10, **caractérisé en ce que** le nettoyage et la stérilisation de l'installation présentent avant l'entrée en fonctionnement normal les étapes suivantes :
le nettoyage des surfaces librement accessibles, reçues dans l'isolateur (1) par l'introduction d'un agent de nettoyage,
le rinçage des surfaces avec de l'eau chaude,
le séchage des surfaces par introduction d'air sec chauffé, et
l'alimentation des surfaces nettoyées et séchées en un moyen de stérilisation.
